(19) Europäisches Patentamt
European Patent Office
Office européen des brevets

(11) EP 3 936 114 A1

(12) EUROPEAN PATENT APPLICATION

(43) Date of publication:
**12.01.2022 Bulletin 2022/02**

(21) Application number: **20305778.1**

(22) Date of filing: **07.07.2020**

(51) Int Cl.:
***A61K 9/00*** *(2006.01)* ***A61K 9/16*** *(2006.01)*
***A61K 9/19*** *(2006.01)* ***A61K 9/50*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Occlugel**
**78350 Jouy en Josas (FR)**

(72) Inventors:
- **BEILVERT, Anne**
**91300 MASSY (FR)**
- **CORUS, Emeline**
**91640 JANVRY (FR)**
- **BEDOUET, Laurent**
**72000 LE MANS (FR)**
- **MOINE, Laurence**
**92210 SAINT CLOUD (FR)**

(74) Representative: **Regimbeau**
**20, rue de Chazelles**
**75847 Paris Cedex 17 (FR)**

(54) **HYDROPHILIC DEGRADABLE MICROSPHERES FOR LOCAL DELIVERING OF GLYCOPEPTIDE ANTIBIOTICS AND POLYCATIONIC PEPTIDE ANTIBIOTICS**

(57) The invention relates to a composition comprising an effective amount of a peptides antibiotic at least one hydrophilic degradable microsphere comprising a crosslinked matrix, and a pharmaceutically acceptable carrier for administration by injection, the crosslinked matrix being based on at least a) between 10 % and 90 % of hydrophilic monomer of general formula (I); b) between 0.1 and 30% mol of a cyclic monomer having an exo-methylene group of formula (II); and c) between 5 % and 90 % of one degradable block copolymer cross-linker, wherein the degradable block copolymer crosslinker is linear or star-shaped and presents ($CH_2$=($CR_{11}$))-groups at all its extremities and wherein the degradable block copolymer crosslinker has a partition coefficient P of between 0.50 and 11.20. The invention also relates to such a composition for use for preventing and/or treating infectious diseases, in particular mammal infectious diseases, by local delivery.

EP 3 936 114 A1

**Description**

## FIELD OF THE INVENTION

The present invention relates to hydrophilic degradable microsphere for local delivering of a peptides antibiotic. In particular, the present invention relates to composition comprising an effective amount of a peptides antibiotic and hydrophilic degradable microspheres. The present invention also relates to said composition for use for preventing and/or treating infectious diseases, in particular mammal infectious diseases, by local delivery.

## TECHNICAL BACKGROUND

To treat bacterial infections, systemic antibiotics are taken orally or intravenously and affect the body as a whole. The choice of initial antibiotic treatment depends on several factors such as the severity of the infection, whether the patient received another antibiotic treatment, or the infection is caused by a microorganism known to be resistant to the usual antibiotics, and the patient's general health. The goal of antibiotic treatment is to stop the infection and prevent its spread.

The systemic chemotherapy approach presents the risk of generating, on the one hand, bacterial resistance, and on the other hand, side effects. Localized therapies are based on a controlled diffusion of the active anti-microbial principles incorporated in slow release devices. This therapeutic alternative makes possible to avoid general effects and obtain high active pharmacological concentrations at the targeted sites. It is essential to maintain in situ a concentration of antibiotics always greater than the minimum inhibitory concentration (MIC). Local antibiotic delivery is useful when infected sites are poorly perfused preventing proper antibiotic infusion. In addition to locally increasing the antibiotic concentration, localized therapies allow the use of antibiotics which are toxics (ototoxicity, nephrotoxicity) when used systemically.

In the art *(*Kanellakopoulou K et al.; Drugs. 2000. 59:1223-32*),* carriers used for the local delivery of antibacterial agents may be classified as non-biodegradable or biodegradable. A major representative of the former category are the polymethylmethacrylate (PMMA) beads often impregnated with gentamicin, vancomycin or tobramycin *(*Kanellakopoulou K et al.; Drugs. 2000 59:1223-32 *; and* Meeker et al., 2019. J Arthroplasty. 34: 1458-61*).* The modern use of local antibiotic in orthopaedics began with the use of polymethylmethacrylate cement impregnated with antibiotics to treat infected joint prostheses. PMMA leads to a rapid release of a high concentration of antibiotic (vancomycin, tobramycin, gentamycin, erythromycin and cefuroxime) during the first several days after implantation. The major disadvantage of this material is the need for their surgical removal at the completion of antibiotic release, which usually takes place 4 weeks after their implantation.

Examples of the latter category (biodegradable) include collagen-gentamicin sponge, apatite-wollastonite glass ceramic blocks, hydroxyapatite blocks, polylactide/polyglycolide implants and the polylactate polymers *(*Kanellakopoulou K et al.; Drugs. 2000. 59:1223-32*).* All of the above systems release antibiotics at high concentrations for the first few days without releasing any antibiotic into the systemic circulation and without producing adverse effects. The biodegradable carriers do not require surgical removal, and of those listed, the collagen-gentamicin sponge has been applied successfully over the last decade for bone infections. Collagen sponges are vector only available on the market loaded with gentamicin. They dissolve entirely in one to two weeks making them therefore unsuitable for filling cavities. In addition, their elution is extremely rapid, mainly on the first day (Sorensen TS et al; 1990.Acta Orthop Scand. 61: 353-6).

Today, there is a need for a biodegradable local delivery system of antibiotics, and more particularly antimicrobial peptides that allows a delivery over an extended period while avoiding a biofilm development on the system as reported for PMMA beads. More particularly, development of novel degradable drug delivery systems (DDS) dedicated for local sustained release of peptide antibiotic participate in the response towards the emergence of multidrug resistance pathogens by reducing the systemic exposition. Use of local DDS loaded with peptides antibiotics could be an efficient tool in the treatment of infections causes by Gram-negative and Gram-positive bacteria in musculoskeletal tissues, skin and soft tissues and the eyes.

Gram-negative bacteria cause infections including pneumonia, bloodstream infections, wound or surgical site infections, and meningitis. Gram-negative bacteria are increasingly resistant to most available antibiotics. These bacteria have developed abilities to find new ways to be resistant and can pass along genetic materials that allow other bacteria to become drug-resistant (Velkov et al; 2016. Future Med Chem. 8: 1017-25).

An original approach to treat infections caused by gram-negative bacteria could be based on the sustained local delivery of peptides antibiotic such peptides belonging to the polymyxin family. Polymyxins are polypeptide antibiotics discovered in 1947 and introduced into the clinic in the late 1950s. Polymyxins are cationic polypeptides that consist of a cyclic heptapeptide possessing a tripeptide side chain acylated at the N-terminal end by a fatty acid tail. Colistin (polymyxin E) and polymyxin B differ by only a single amino acid in the peptide ring, with a phenylalanine in polymyxin B and a leucine in colistin (Poirel et al; 2017.Clin Microbiol Rev. 30:557-96). Polymyxins neutralize the Gram-negative

bacteria due to their high affinity for lipopolysaccharides (LPS) by means of electrostatic interactions between the negatively charged LPS (phosphate moieties of the lipid A region of LPS) and the positively charged amino-butyric residues of polymyxins. Parenteral administration of the polymyxins led to nephrotoxicity and their clinical use decreased by the 1970s. Over the last two decades there was a renewed interest in polymyxins according to the emergence of multidrug-resistant bacteria, notably among *Pseudomonas aeruginosa* and *Acinetobacter baumannii* and the absence of new antibiotics effective on these bacteria in the future. This situation should favor the rebirth of the polymyxin antibiotics, which represent an important last-line of defence against gram-negative pathogens. Modifications of the polymyxin structure to reduce the renal toxicity but preserving the bactericidal activity have failed (Velkov et al; 2016. Future Med Chem. 8: 1017-25). Until now, polymyxins were immobilized on cellulose microspheres to bind the circulating LPS for clinical blood purification during septic shock (Stegmayr. 2001.Ther Apher.5:123-7). Polymyxins were encapsulated in PLGA microspheres to maintain a slow release of polymyxin in the blood of mice to protect them against endotoxin-induced sepsis (Nanjo et al; 2013. Journal of Infection and Chemotherapy. 19: 683-90). No local DDS of polymyxins seems to have been developed until now to cure local Gram-negative bacteria. Loading of polymyxins on a tuneable degradable carrier to treat locally infections caused by Gram-negative bacteria resistant to systemic antibiotics would be an innovative anti-infective strategy.

Infections caused by multidrug-resistant Gram-positive bacteria represent a major public health burden. Gram-positive bacteria are among the most common causes of infection, and the prevalence of drug-resistant strains of Gram-positive bacteria (including methicillin-resistant *Staphylococcus aureus* (MRSA) and glycopeptide-resistant enterococci) is increasing (Munita et al; 2015. Clin Infect Dis. 61(Suppl 2): S48-S57). Gram-positive organisms (bacteria of the genera Staphylococcus, Streptococcus and Enterococcus) are among the most common bacterial causes of clinical infections. MRSA is a pathogen of concern due to its resistance to almost all B-lactam antimicrobials (i.e. penicillins, cephalosporins and carbapenems). Antimicrobial peptides, in particular the glycopeptides (Vancomycin, Teicoplanin, Daptomycin, Telavancin, dalbavancin, Oritavancin) represent a major class of molecules effective towards gram-positive bacteria. Glycopeptides kill Gram-positive bacteria by inhibiting the cross-linking stabilisation step in bacterial cell wall formation. They constitute an alternative to the penicillins in cases of resistance or intolerance. Vancomycin is the second most widely used antibiotic, showing antimicrobial activity against most Gram-positive germs, including MRSA. Teicoplanin (produced by the actinomycete *Actinoplanes teichomyceticus*) is structurally similar to vancomycin and is a complex of five closely related glycopeptides that have the same heptapeptide base and an aglycone that contains aromatic amino acids, one $\alpha$-D-mannose and two N-acetyl-D-glucosamine residues, one of which is substituted with an acyl moiety composed of a fatty acid residue containing 10 or 11 carbon atoms. Teicoplanin is about five times as lipophilic as vancomycin.

Unfortunately, resistance to glycopeptides is developing among Gram-positive pathogenic bacteria, resistance to vancomycin in *Staphylococcus aureus* (Gardete et Tomasz; 2014. 124: 2836-40) and teicoplanin in *Staphylococcus epidermidis* (Trueba et al; 2006. J Clin Microbiol. 44:1922-23). One way to reduce the emergence of resistance to these antibiotics of last resort is to privilege when it is possible a local therapy instead of a systemic treatment. Local delivery of glycopeptides loaded on degradable carriers represents an opportunity to treat locally serious infections while reducing the risk to create resistance which is induced during systemic treatments.

Many DDS of teicoplanin are described for local delivery in the treatment of chronic osteomyelitis. Jia et al. 2010 (Acta Biomaterialia. 6: 812-19) prepared composite materials composed of borate bioactive glass and chitosan. *In vitro,* $\approx$ 40 % of teicoplanin release occurred in 24 h and $\approx$ 70 % after 7 days. *In vivo* after 12 weeks, the implants are efficient to treat osteomyelitis in a rabbit model by providing teicoplanin sustained release. Teicoplanin was encapsulated in poly(ethylene glycol) monomethyl ether (mPEG) and poly(lactic-co-glycolic acid) (PLGA) copolymer as a sol-gel drug delivery system for treating bone infection. Gelification of composition occurred in the target tissues and was effective to treat experimental osteomyelitis induced in rabbit (Peng et al; 2010. Biomaterials. 31: 5227-36). Calcium sulfate pellets containing teicoplanin were also prepared. The *in vitro* drug elution lasts for 1 week (80 % of release). *In vivo,* 6 weeks after implantation in a rabbit model of osteomyelitis, a reduction of bacterial infection and bone repair were observed (Jia et al; 2010. Antimicrob Agents Chemother. 54: 170-176). Teicoplanin was encapsulated in PLGA microspheres for intra-articular implantation in rabbit. Efficient concentration of teicoplanin in synovial fluid was measured for 5 weeks (Yenice et al. 2003. Journal of Microencapsulation: 20: 705-17). An *in vitro* sustained delivery of teicoplanin (37 % in 100 h) was obtained with tripolyphosphate cross-linked chitosan nanoparticles containing the drug (Kahdestani et al; 2020. Polym. Bull. 20). As for gelatin sponges, the teicoplanin impregnation is poor compared to vancomycin and are not considered as promising carriers for the local application of teicoplanin to infected wounds (Drognitz et al; 2006. Infection 34(1):29-34). Local delivery of teicoplanin by means of different degradable carriers is an efficient strategy in animal model to treat experimental osteomyelitis.

To locally treat infections caused by Gram-positive and Gram-negative bacteria, and to avoid the emergence of resistance due to systemic anti-biotherapy, the inventors have noticed the interest represented by the local antibiotic delivery systems. There is a need of a degradable carrier of antimicrobial peptides effective against the Gram-negative and Gram-positive germs that allows a sustained release without burst and that allows an easy and quick loading of the active ingredients. The inventors have also noticed the absence of degradable carriers for local delivery of polymyxins

into wounds.

## SUMMARY OF THE INVENTION

The inventors surprisingly found that degradable hydrophilic microspheres composed of a crosslinked hydrogel display strong affinity for peptides antibiotics, in particular for both polymyxins and teicoplanin peptides, allowing their efficient loading in few minutes onto preformed microspheres and their subsequent sustained release.

The present invention offers the possibility to simplify the process for the local delivery of different antimicrobial peptides in infected tissues, i.e by simply mixing antimicrobial peptides in solution in water with preformed degradable microspheres, a simpler procedure than the preparation of PMMA beads impregnated with antibiotics (Meeker et al., 2019. J Arthroplasty 34: 1458-61)

The inventors have discovered hydrophilic degradable microspheres that may be used as biocompatible drug carrier for local delivery and that present affinity with polycationic peptides which may have a fatty acid tail, including polymyxins, and glycopeptide antibiotics having a fatty acid tail, including teicoplanin.

The inventors have thus discovered a local delivery system of antimicrobial peptides. The delivery system is free of organic solvent with a tuneable degradation time ranging from day to months, that is easy to load (in water, at room temperature in few minutes), that allows a complete drug release and that avoids intense inflammatory reaction and immediate burst.

The local delivery system of the invention allows an extended and local release of antimicrobial peptides. Such a delivery avoids the release of any antibiotic in the systemic circulation and thus the adverse effects while allowing an extended duration of release without the need of several administration and without surgical removal of the delivery system.

In a first aspect, the invention relates to a composition comprising an effective amount of a peptides antibiotic, at least one hydrophilic degradable microsphere comprising a crosslinked matrix, and a pharmaceutically acceptable carrier for administration by injection, the crosslinked matrix being based on at least:

a) between 10 % and 90 % of hydrophilic monomer of general formula (I):

$$(CH_2=CR_1)\text{-CO-D} \qquad (I)$$

wherein:

- D is O-Z or NH-Z, Z being $(C_1\text{-}C_6)$alkyl group, $-(CR_2R_3)_m\text{-}CH_3$, $-(CH_2\text{-}CH_2\text{-}O)_m\text{-}H$, $(CH_2\text{-}CH_2\text{-}O)_m\text{-}CH_3$, $-C(R_4OH)m$ or $-(CH_2)_m\text{-}NR_5R_6$ with m being an integer from 1 to 30;
- $R_1$, $R_2$, $R_3$, $R_4$, $R_5$ and $R_6$ are, independently of one another, hydrogen atom or a $(C_1\text{-}C_6)$alkyl group;

b) between 0.1 and 30% mol of a cyclic monomer having an exo-methylene group of formula (II):

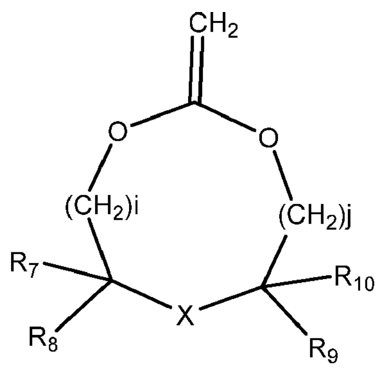
(II)

wherein:

- $R_7$, $R_8$, $R_9$ and $R_{10}$ are, independently of one another, hydrogen atom, a $(C_1\text{-}C_6)$alkyl group or an aryl group;
- i and j are independently of one another an integer chosen between 0 and 2; and
- X is a single bond or an oxygen atom; and

c) between 5 % and 90 % of one degradable block copolymer cross-linker, wherein the degradable block copolymer crosslinker is linear or star-shaped and presents ($CH_2$=($CR_{11}$))-groups at all its extremities, each $R_{11}$ being independently of one another hydrogen atom or a ($C_1$-$C_6$)alkyl group, and wherein the degradable block copolymer crosslinker has a partition coefficient P of between 0.50 and 11.20, or a hydrophobic/hydrophilic balance R between 1 and 20;

the percentages of the monomers a) to c) being expressed in moles relative to the total number of moles of the monomers.

In a second aspect, the invention relates to the composition of the invention, for use for preventing and/or treating infectious diseases, in particular mammal infectious diseases, by local delivery.

In a third aspect, the invention relates to the hydrophilic degradable microsphere of the invention for use for locally delivering an effective amount of a peptides antibiotic to a subject in need thereof.

In a fourth aspect, the invention relates to a pharmaceutical kit comprising:

i) at least one hydrophilic degradable microsphere of the invention in association with a pharmaceutically acceptable carrier for administration by injection;
ii) an effective amount of peptides antibiotic; and
iii) optionally an injection device,

the hydrophilic degradable microsphere and the peptides antibiotic being packed separately.

## DETAILED DESCRIPTION OF THE INVENTION

*Definition*

In the context of the invention, by the expression "matrix based on" is meant a matrix comprising the mixture and/or the product of the reaction between the starting components used for the heterogeneous medium polymerisation of this matrix, preferably only the product of the reaction between the different starting components used for this matrix, some of which may be intended to react or may react with each other or with their close chemical environment, at least in part, during the different phases of the process of manufacture of the matrix, in particular during a polymerisation stage.

Thus, the starting components are the reagents intended to react together during the polymerization of the matrix. The starting components are therefore introduced into a reaction mixture optionally additionally comprising a solvent or a mixture of solvents and/or other additives such as at least one salt and/or at least one polymerization initiator and/or at least one stabilizer such as PVA.

In the context of the present invention, the reaction mixture comprises at least the monomers a), b), c) as defined in the claims and in this description, optionally a polymerization initiator such as, for example, t-butyl peroxide, benzoyl peroxide, azobiscyanovaleric acid (also called 4,4'-Azobis(4-cyanopentanoic acid)), AIBN (azobisisobutyronitrile), or 1,1'- Azobis(cyclohexane carbonitrile) or one or more photo-initiators such as 2-Hydroxy-4'-(2-hydroxyethoxy)-2-methylpropiophenone (106797-53-9); 2-Hydroxy-2-methylpropiophenone (Darocur® 1173, 7473-98-5); 2,2-Dimethoxy-2-phenylacetophenone (24650-42-8); 2,2-Dimethoxy-2-phenyl acetophenone (Irgacure®, 24650-42-8) or 2-Methyl-4'-(methylthio)-2-morpholinopropiophenone (Irgacure®, 71868-10-5), and at least one solvent, preferably a solvent mixture comprising an aqueous solvent and an organic solvent such as an apolar aprotic solvent, for example a water/toluene mixture.

Thus, according to the present invention, the matrix is at least based on the monomers a), b) and c) as defined in the claims and in this description, these compounds thus being starting components.

Thus, in the present description, expressions such as "the [starting component X] is added to the reaction mixture in an amount of between YY% and YYYY%" and "the cross-linked matrix is based on [starting component X] in an amount of between YY% and YYYY%" are interpreted in a similar manner. Similarly, expressions such as "the reaction mixture comprises at least [starting component X]" and "the cross-linked matrix is based on at least [starting component X]" are interpreted in a similar manner.

In the context of the invention, "organic phase" of the reaction mixture means the phase comprising the organic solvent and the compounds soluble in said organic solvent, in particular the monomers, and the polymerization initiator.

In the context of the invention, the terms "($C_X$-$C_Y$)alkyl group" mean a saturated monovalent hydrocarbon chain, linear or branched, containing X to Y carbon atoms, X and Y being integers between 1 and 36, preferably between 1 and 18, in particular between 1 and 6. Examples are methyl, ethyl, propyl, isopropyl, butyl, isobutyl, sec-butyl, tert-butyl, pentyl or hexyl groups.

In the context of the invention, the terms "aryl group" and "($C_X$-$C_Y$)aryl" mean an aromatic hydrocarbon group, preferably having X to Y carbon atoms, and comprising one or more adjacent rings, X and Y being integers between 5 and 36, preferably between 5 and 18, in particular between 5 and 10. Examples are phenyl or naphthyl groups.

In the context of the invention, the terms "partition coefficient P" mean the ratio of concentrations of a compound in a mixture of two immiscible solvents at equilibrium: water and 1-octanol. This ratio is therefore a comparison of the solubilities of the solute in these two liquids. Hence the octanol/water partition coefficient measures how hydrophilic (octanol/water ratio < 1) or hydrophobic (octanol/water > 1) a compound is. Partition coefficient P may be determined by measuring the solubilities of the compound in water and in 1-octanol and by calculating the ratio solubility in octanol / solubility in water. Partition P may also be determined *in silico.*

In the context of the invention, the hydrophobic/hydrophilic balance R of the degradable crosslinkers is quantified by the ratio of the number of hydrophobic units to the number of hydrophilic units according the following equation:

$$R = \frac{N_{hydrophobic\ units}}{N_{hydrophilic\ units}}$$

with N being an integer and representing the number of unit(s).

For example, for the crosslinkers that can be used in the present invention, R is:

$$R = \frac{N_{CHlactide} + N_{CH3lactide} + N_{CH2glycolide} + 5 \times N_{CH2caprolactone}}{N_{EO\ unit}}$$

with N being an integer and representing the number of unit(s).

In the context of the invention, the expression "between X and Y" (wherein X and Y are numerical value) means a range of numerical values in which the limits X and Y are inclusive.

In the context of the invention, the terms "degradable microsphere" mean that the microsphere is degraded or cleaved by hydrolysis in a mixture of degradation products composed of low-molecular-weight compounds and water-soluble polymer chains having molecular weights below the threshold for renal filtration of 50 kg $mol^{-1}$.

In the context of the invention, the expression "immediate release (IR)" of an active ingredient means the rapid release of the active ingredient from the formulation to the location of delivery as soon as the formulation is administered.

In the context of the invention, the expression "extended-release" of an active ingredient means either the "sustained-release (SR)" or the "controlled-release (CR)" of active ingredients from the formulation to the location of delivery at a predetermined rate for an extended period of time and maintaining a constant active ingredient level for this period of time with minimum side effects. The controlled-release (CR) differs from the sustained-release (SR) in that CR maintains drug release over a sustained period at a constant rate whereas SR maintains drug release over a sustained period but not at a constant rate.

In the context of the invention, the expression "sustained-release" of an active ingredients means an extended-release (as defined above) of an active ingredient from the formulation to the location of delivery in order to maintain for a certain predetermined time the drug in tissue of interest at therapeutic concentrations by means of an initial dose portion.

In the context of the invention, the expression "controlled-release (CR)" of an active ingredient means an extended-release (as defined above) of an active ingredient from the formulation to the location of delivery that provides some control of temporal or spatial nature, or both.

For the purpose of the invention, the term "pharmaceutically acceptable" is intended to mean what is useful to the preparation of a pharmaceutical composition, and what is generally safe and non toxic, for a pharmaceutical use.

The term « pharmaceutically acceptable salt » is intended to mean, in the framework of the present invention, a salt of a compound which is pharmaceutically acceptable, as defined above, and which possesses the pharmacological activity of the corresponding compound. Such salts comprise:

(1) hydrates and solvates,

(2) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and

(3) salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

*Microsphere*

According to the present invention, the hydrophilic degradable microsphere comprises a crosslinked matrix that is based on at least:

a) between 10 % and 90 % of hydrophilic monomer of general formula (I):

$$(CH_2=CR_1)\text{-}CO\text{-}D \qquad (I)$$

wherein:

- D is O-Z or NH-Z, Z being $(C_1\text{-}C_6)$alkyl group, $-(CR_2R_3)_m\text{-}CH_3$, $-(CH_2\text{-}CH_2\text{-}O)_m\text{-}H$, $(CH_2\text{-}CH_2\text{-}O)_m\text{-}CH_3$, $-C(R_4OH)m$ or $-(CH_2)_m\text{-}NR_5R_6$ with m being an integer from 1 to 30;
- $R_1$, $R_2$, $R_3$, $R_4$, $R_5$ and $R_6$ are, independently of one another, hydrogen atom or a $(C_1\text{-}C_6)$alkyl group;

b) between 0.1 and 30% mol of a cyclic monomer having an exo-methylene group of formula (II):

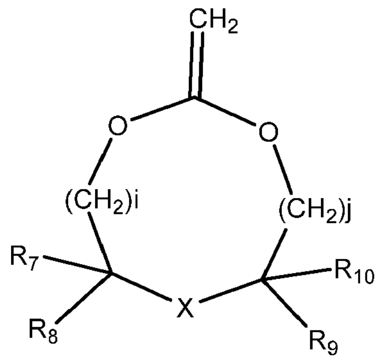

(II)

wherein:

- $R_7$, $R_8$, $R_9$ and $R_{10}$ are, independently of one another, hydrogen atom, a $(C_1\text{-}C_6)$alkyl group or an aryl group;
- i and j are independently of one another an integer chosen between 0 and 2; and
- X is a single bond or an oxygen atom;

and

c) between 5 % and 90 % of one degradable block copolymer cross-linker, wherein the degradable block copolymer crosslinker is linear or star-shaped and presents $(CH_2=(CR_{11}))$-groups at all its extremities, each $R_{11}$ being independently of one another hydrogen atom or a $(C_1\text{-}C_6)$alkyl group, and wherein the degradable block copolymer crosslinker has a partition coefficient P of between 0.50 and 11.20, or a hydrophobic/hydrophilic balance R between 1 and 20;

the percentages of the monomers a) to c) being expressed in moles relative to the total number of moles of the monomers.

The components a) the hydrophilic monomer of general formula (I), b) the cyclic monomer having an exo-methylene group of formula (II), and c) the degradable block copolymer cross-linker, are thus starting components of the crosslinked matrix.

In the context of the invention, the hydrophilic degradable microsphere is a swellable degradable (i.e. hydrolyzable) cross-linked polymer in the form of spherical particle having a diameter after swelling ranging between 20 μm and 1200 μm. In particular the polymer of the invention is constituted of at least one chain of polymerized monomers a), b) and c) as defined above.

In the context of the invention, a polymer is swellable if it has the capacity to absorb liquids, in particular water. The expression "size after swelling" means thus that the size of the microspheres is considered after the polymerization and sterilization steps that take place during their preparation.

Advantageously, the microsphere of the invention has a diameter after swelling of between 20 μm and 100 μm, 40 μm and 150 μm, 100 μm and 300 μm, 300 μm and 500 μm, 500 μm and 700 μm, 700 μm and 900 μm or 900 μm and 1200 μm, as determined by optical microscopy. Microspheres are advantageously small enough in diameter to be injected through needles, catheters or microcatheters with internal diameters ranging from a few hundred micrometres

to more than one millimetres.

In the context of the invention, the terms "hydrophilic monomer" mean a monomer having a high affinity for water, i.e. tending to dissolve in water, to mix with water, to be wetted by water, or capable of swelling in water after polymerization.

The hydrophilic monomer a) is of general formula (I):

$$(CH_2{=}CR_1)\text{-}CO\text{-}D \qquad (I)$$

wherein:

- D is O-Z or NH-Z, Z being $(C_1\text{-}C_6)$alkyl group, $-(CR_2R_3)_m\text{-}CH_3$, $-(CH_2\text{-}CH_2\text{-}O)_m\text{-}H$, $(CH_2\text{-}CH_2\text{-}O)_m\text{-}CH_3$, $-C(R_4OH)m$ or $-(Ch_2)_m\text{-}NR_5R_6$ with m being an integer from 1 to 30;
- $R_1$, $R_2$, $R_3$, $R_4$, $R_5$ and $R_6$ are, independently of one another, hydrogen atom or a $(C_1\text{-}C_6)$alkyl group.

Advantageously, the hydrophilic monomer a) is selected from the group consisting of sec-butyl acrylate, n-butyl acrylate, t-butyl acrylate, t-butyl methacrylate, methylmethacrylate, N-dimethylaminoethyl(methyl)acrylate, N,N-dimethylaminopropyl-(meth)acrylate, t-butylaminoethyl (methyl)acrylate, N,N-diethylaminoacrylate, acrylate terminated poly(ethylene oxide), methacrylate terminated poly(ethylene oxide), methoxy poly(ethylene oxide) methacrylate, butoxy poly(ethylene oxide) methacrylate, acrylate terminated poly(ethylene glycol), methacrylate terminated poly(ethylene glycol), methoxy poly(ethylene glycol) methacrylate, butoxy poly(ethylene glycol) methacrylate; advantageously acrylate terminated poly(ethylene glycol), methacrylate terminated poly(ethylene glycol), methoxy poly(ethylene glycol) methacrylate, butoxy poly(ethylene glycol) methacrylate.

More advantageously, the hydrophilic monomer a) is poly(ethylene glycol) methyl ether methacrylate.

In the context of the invention, the hydrophilic monomer a) is advantageously present in the reaction mixture in an amount of between 10 % and 90%, preferably from between 30% and 80 % by mole, relative to the total number of moles of the monomers.

In the context of the invention, component b) is a cyclic monomer having an exo-methylene group of formula (II) as defined above, wherein:

- $R_7$, $R_8$, $R_9$ and $R_{10}$ are, independently of one another, hydrogen atom, a $(C_1\text{-}C_6)$alkyl group or an aryl group;
- i and j are independently of one another an integer chosen between 0 and 2;
- X is a single bond or an oxygen atom.

Advantageously, component b) is a cyclic monomer having an exo-methylene group of formula (II) as defined above, wherein:

- $R_7$, $R_8$, $R_9$ and $R_{10}$ are, independently of one another, hydrogen atom or a $(C_5\text{-}C_7)$aryl group;
- i and j are independently of one another an integer chosen between 0 and 2;
- X is a single bond or an oxygen atom.

Advantageously, component b) is a cyclic monomer having an exo-methylene group of formula (II) as defined above, wherein:

- $R_7$, $R_8$, $R_9$ and $R_{10}$ are, independently of one another, hydrogen atom or a $(C_5\text{-}C_7)$aryl group;
- i and j are independently of one another an integer chosen between 0 and 1;
- X is a single bond or an oxygen atom.

Advantageously, the component b) is selected from the group consisting of 2-methylene-1,3-dioxolane, 2-methylene-1,3-dioxane, 2-methylene-1,3-dioxepane, 2-Methylene-1,3,6-Trioxocane and derivatives thereof, in particular benzo derivatives and phenyl substituted derivatives, advantageously from the group consisting of 2-methylene-1,3-dioxolane, 2-methylene-1,3-dioxane, 2-methylene-1,3-dioxepane, 2-methylene-4-phenyl-1,3-dioxolane, 2-Methylene-1,3,6-Trioxocane and 5,6-benzo-2-methylene-1,3dioxepane, more advantageously from the group consisting of 2-methylene-1,3-dioxepane, 5,6-benzo-2-methylene-1,3dioxepane and 2-Methylene-1,3,6-Trioxocane. More advantageously, the component b) is 2-methylene-1,3-dioxepane or 2-Methylene-1,3,6-Trioxocane.

In the context of the invention, the cyclic monomer b) having an exo-methylene group of general formula (II) is advantageously present in the reaction mixture in an amount of between 0.1 % and 30 %mol, preferably from between 1% and 20 %, and in particular between 1% and 10% by mole, relative to the total number of moles of the monomers.

In the context of the invention, component c) is a degradable block copolymer crosslinker, wherein the degradable block copolymer crosslinker is linear or star-shaped and presents ($CH_2$=($CR_{11}$)-groups at all its extremities, each $R_{11}$ being independently of one another hydrogen atom or a ($C_1$-$C_6$)alkyl group.

In the context of the invention, the degradable block copolymer crosslinker has a partition coefficient P of between 0.50 and 11.20, advantageously between 2.00 and 9.00; or the degradable block copolymer crosslinker has a hydrophobic/hydrophilic balance R between 1 and 20, advantageously between 3 and 15.

The hydrophobicity of the crosslinker and its concentration, and thus the partition coefficient P of the crosslinker, influences the release of the peptides antibiotics. Thus, when the partition coefficient P increases, the delivery time of peptides antibiotic, in particular of polycationic peptides antibiotics also increases. The concentration of the crosslinker also influences the release of peptides antibiotics, in particular of hydrophobic glycopeptides antibiotics, high concentration of crosslinker prevents the immediate release of a large part of the peptides antibiotics, and lengthens the release time.

As intended therein, the expression "copolymer cross-linker" is intended to mean that the copolymer contains a functional group containing a double bond at least two of its extremities in order to link together several polymer chains.

The cross-linker c) as defined above is linear or star-shaped (advantageously from 3 to 8 arms) and it presents ($CH_2$=($CR_{11}$))-groups at all its extremities, each $R_{11}$ being independently of one another hydrogen atom or a ($C_1$-$C_6$)alkyl group. Advantageously, the crosslinker c) presents ($CH_2$=($CR_{11}$))-CO- or ($CH_2$=($CR_{11}$))-CO-O groups at all its extremities, each $R_{11}$ being independently of one another hydrogen atom or a ($C_1$-$C_6$)alkyl group. Advantageously, the $R_{11}$ are identical and are H or a ($C_1$-$C_6$)alkyl group.

Advantageously, the cross-linker c) as defined above is linear and it presents ($CH_2$=($CR_{11}$))-groups at both its extremities, each $R_{11}$ being independently of one another hydrogen atom or a ($C_1$-$C_6$)alkyl group. Advantageously, the crosslinker c) presents ($CH_2$=($CR_{11}$))-CO- or ($CH_2$=($CR_{11}$))-CO-O groups at both its extremities, each $R_{11}$ being independently of one another hydrogen atom or a ($C_1$-$C_6$)alkyl group. Advantageously, the $R_{11}$ are identical and are H or a ($C_1$-$C_6$)alkyl group.

Advantageously, the crosslinker c) is of general formula (IIIa), (IIIb) or (IIIc) as follows:

$$(CH_2{=}CR_{11})\text{-}CO\text{-}X_n\text{-}PEG_p\text{-}X_k\text{-}CO\text{-}(CR_{11}{=}CH_2) \qquad \text{(IIIa);}$$

$$(CH_2{=}CR_{11})\text{-}CO\text{-}O\text{-}X_n\text{-}PEG_p\text{-}X_k\text{-}O\text{-}CO\text{-}(CR_{11}{=}CH_2) \qquad \text{(IIIb)}$$

$$PEG_p\text{-}(X_n\text{-}O\text{-}CO\text{-}(CR_{11}{=}CH_2))_z \qquad \text{(IIIc);}$$

wherein:

- each $R_{11}$ is independently of one another hydrogen atom or a ($C_1$-$C_6$)alkyl group;
- X independently represents PLA, PGA, PLGA, PCL or PLAPCL;
- n, k and p respectively represent the degree of polymerization of X, and PEG, n and k independently being integers from 1 to 150, and p being an integer from 1 to 100;
- z represent the number of arms of the PEG molecule being integers from 3 to 8.

Advantageously, when the crosslinker c) is of general formula (IIIc), n may be identical or different in each arm of the PEG.

In the context of the invention:

- PEG means polyethylene glycol of formula (IVa):

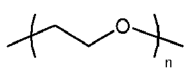

(IVa);

- PLA means poly-lactic acid (also named poly-lactide) of formula (IVb):

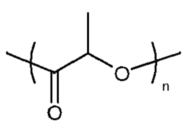

(IVb);

- PGA means poly-glycolic acid (also named poly-glycolide) of formula (IVc):

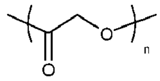
(IVc);

- PLGA means poly-lactic-glycolic acid and comprises both lactide and glycolide units, the degree of polymerization is the sum of the number of lactide and glycolide units; and
- PCL means poly(caprolactone) of formula (IVd):

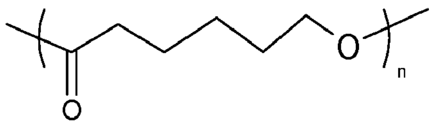
(IVd;

and

- PLAPCL means poly-lactic acid poly-caprolactone and comprises both lactide and caprolactone units,.

Advantageously, the crosslinker c) is of general formula (IIIa), (IIIb) or (IIIc), in particular (IIIa) or (IIIb), as defined above, wherein X represents PLAPCL or PCL. More advantageously, the crosslinker c) is of general formula (IIIa), (IIIb) or (IIIc), in particular (IIIa) or (IIIb), wherein X represents PCL.

Advantageously, the crosslinker c) is of general formula (IIIa), (IIIb) or (IIIc), in particular (IIIa) or (IIIb), as defined above, wherein n and k independently being integers from 1 to 150, and p being an integer from 1 to 100.

Advantageously, the crosslinker c) is of general formula (IIIa), (IIIb) or (IIIc), in particular (IIIa) or (IIIb), as defined above, wherein the $R_{11}$ are identical and are H or a $(C_1\text{-}C_6)$alkyl group.

Advantageously, the crosslinker c) is selected from the group consisting of compounds of general formula (IIIa), (IIIb) or (IIIc), in particular (IIIa) or (IIIb), as defined above, wherein:

- X= PLA, n + k=12 and p=13 (such as PEG13-PLA12);
- X= PLAPCL, n + k=10 and p=13 (such as PEG13-LA8-Cl2 or PEG13-LA7-Cl3);
- X = PLAPCL, n + k= 9 and p=13 (such as PEG13-LA4-Cl5);
- X = PLAPCL, n + k= 8 and p=13 (such as PEG13-LA2-Cl6);
- X = PCL; n + k = 8 and p=13 (such as PEG13-PCl8);
- X =PCL, n + k = 10 and p=4 (such as PEG4-PCL10); or
- X =PCL, n + k = 12 and p=2 (such as PEG2-PCL12).

Within the definitions of the crosslinker c) above, the polyethylene glycol (PEG) has a length of 100 to 10 000 g/mol, preferably 100 to 2 000 g/mol, more preferably 100 to 1 000 g/mol

In the context of the invention, the crosslinker c) is advantageously present in the reaction mixture in an amount of between 5 % and 90 %mol, preferably from between 5% and 60 %, and in particular between 10% and 60% by mole, relative to the total number of moles of the monomers. Advantageously, when the composition of the invention comprises glycopeptides antibiotic such as teicoplanin as peptides antibiotic, the concentration of the crosslinker influences its release. For amount greater than 10%, an optimal release of the glycopeptides antibiotic such as teicoplanin is achieved, in particular because it prevents the immediate release of a large part of the glycopeptides antibiotic. Therefore, when the composition of the invention comprises glycopeptides antibiotic such as teicoplanin as peptides antibiotic, the crosslinker c) is advantageously present in the reaction mixture in an amount of between 5 % and 90 %mol, preferably from between 5% and 60 %, and in particular between 10% and 60% by mole, relative to the total number of moles of the monomers.

Advantageously, when the composition of the invention comprises polycationic peptides antibiotic such as polymixin B as peptides antibiotic, the hydrophobicity of the crosslinker and thus the partition coefficient P of the crosslinker or the hydrophobic/hydrophilic balance R influences its release. For partition coefficient P of between 0.50 and 11.20, advantageously between 2.00 and 9.00, or hydrophobic/hydrophilic balance R between 1 and 20, preferentially between 3 and 15, an optimal release of the polycationic peptides antibiotic such as polymixin B is achieved, in particular because it prevents the immediate release of a large part of the polycationic peptides antibiotic.

According to the invention, the crosslinked matrix of the hydrophilic degradable microsphere is advantageously further based on a chain transfer agent d).

For the purposes of this invention, "transfer agent" means a chemical compound having at least one weak chemical bond. This agent reacts with the radical site of a growing polymer chain and interrupts the growth of the chain.

In the chain transfer process, the radical is temporarily transferred to the transfer agent which restarts growth by transferring the radical to another polymer or monomer.

Advantageously, the chain transfer agent d) is selected from the group consisting of monofunctional or polyfunctional thiols, alkyl halides, transition metal salts or complexes and other compounds known to be active in free radical chain transfer processes such as 2,4-diphenyl-4-methyl-1-pentene. More advantageously, the chain transfer agent is a cycloaliphatic or aliphatic, thiol preferably having from 2 to 24 carbon atoms, more preferably between 2 an 12 carbon atoms, and having or not a further functional group selected from the groups amino, hydroxy and carboxy. Advantageously, the chain transfer agent d) is selected from the group consisting of thioglycolic acid, 2-mercaptoethanol, dodecane thiol and hexane thiol.

According to the invention, the chain transfer agent is advantageously present in the reaction mixture in an amount of, for example, from 0.1 to 10%, preferably from 2 to 5 % by mole, relative to the number of moles of monomer a).

In a particular aspect of the invention, the crosslinked matrix is only based on starting components a), b), c) and optionally d), as defined above and in the contents abovementioned, no other starting component are thus added to the reaction medium. It is thus clear that the sum of the above-mentioned contents of monomers (a), (b) and (c) must be equal to 100 %.

According to another aspect of the invention, the crosslinked matrix is advantageously further based on at least one ionised or ionisable monomer e) of general formula (V):

$$(CH_2{=}CR_{12})\text{-}M\text{-}E \qquad (V),$$

wherein:

- $R_{12}$ is hydrogen atom or a $(C_1\text{-}C_6)$alkyl group;
- M is a single bond or a divalent radical having 1 to 20 carbon atoms, advantageously a single bond;
- E is a ionised or ionisable group being advantageously selected from the group consisting of - COOH, -COO-, $-SO_3H$, $-SO_3^-$, $-PO_4H_2$, $-PO_4H^-$, $-PO_4^{2-}$, $-NR_{13}R_{14}$, and $-NR_{15}R_{16}R_{17}^+$; $R_{13}$, $R_{14}$, $R_{15}$, $R_{16}$ et $R_{17}$ being independently of one another hydrogen atom or a $(C_1\text{-}C_6)$alkyl group.

In the context of the invention, an ionised or ionisable group is understood to be a group which is charged or which may be in charged form (in the form of an ion), i.e. which carries at least one positive or negative charge, depending on the pH of the medium. For example, the COOH group may be ionised in the $COO^-$ form, and the $NH_2$ group may be ionised in the form of $NH_3^+$.

The introduction of an ionised or ionisable monomer into the reaction media makes it possible to increase the hydrophilicity of the resulting microspheres, thereby increasing the swelling rate of said microspheres, further facilitating their injection via catheters and microcatheters. In addition, the presence of an ionised or ionisable monomer improves the loading of active substances into the microsphere.

In an advantageous embodiment, the ionised or ionisable monomer e) is a cationic monomer, advantageously selected from the group consisting of -(methacryloyloxy)ethyl phosphorylcholine, 2-(dimethylamino)ethyl (meth)acrylate, 2-(diethylamino)ethyl (meth)acrylate and 2-((meth)acryloyloxy)ethyl] trimethylammonium chloride, more advantageously the cationic monomer is diethylamino)ethyl (meth)acrylate. Advantageously, the ionised or ionisable monomer e) is present in the reaction mixture in an amount of between 0 % and 30 % by mole, advantageously between 1 % and 30 % by mole, preferably from between 10% and 15 % by mole, relative to the total number of moles of the monomers.

In another advantageous embodiment, the ionised or ionisable monomer e) is an anionic monomer advantageously selected from the group consisting of acrylic acid, methacrylic acid, 2-carboxyethyl acrylate, 2-carboxyethyl acrylate oligomers, 3-sulfopropyl (meth)acrylate potassium salt and 2-(methacryloyloxy)ethyl]dimethyl-(3-sulfopropyl)ammonium hydroxide, more advantageously, the anionic monomer is acrylic acid. Advantageously, the ionised or ionisable monomer e) is present in the reaction mixture in an amount of between 0 % and 50 % by mole, advantageously between 1 % and 50 % by mole, preferably from between 10% and 30 % by mole, relative to the total number of moles of the monomers.

Advantageously, the ionised or ionisable monomer e) is acrylic acid and is advantageously present in the reaction mixture in an amount of between 0 % and 30 % by mole, advantageously between 1 % and 30 % by mole, preferably from between 10% and 30 % by mole, relative to the total number of moles of the monomers.

The microsphere of the invention can be readily synthesized by numerous methods well-known to the one skilled in the art. By way of example, the microsphere of the invention can be obtained by direct or inverse suspension polymerization as described below and, in the Examples, or by microfluidic.

A direct suspension may proceed as follows:

(1) stirring or agitating a mixture comprising

(i) at least the starting components a), b) and c) as defined above;
(ii) a polymerization initiator present in amounts ranging from 0.1 to approximately 2 parts per weight per 100 parts by weight of the monomers;
(iii) a surfactant in an amount no greater than about 5 parts by weight per 100 parts by weight of the monomers, preferably no greater than about 3 parts by weight and most preferably in the range of 0.5 to 1.5 parts by weight; and
(iv) water to form an oil in water suspension; and

(2) polymerizing the starting components.

In such a direct suspension polymerization, the surfactant may be selected from the group consisting of hydroxyethylcellulose, polyvinyl alcohol (PVA), polyvinylpyrrolidone, polyethylene oxide, polyethylene glycol and Polysorbate 20 (Tween® 20).

An inverse suspension may proceed as follows:

(1) stirring or agitating a mixture comprising:

(i) at least the starting components a), b) and c) as defined above;
(ii) a polymerization initiator present in amounts ranging from 0.1 to approximately 2 parts per weight per 100 parts by weight of the monomers;
(iii) a surfactant in an amount no greater than about 5 parts by weight per 100 parts by weight of the monomers, preferably no greater than about 3 parts by weight and most preferably in the range of 0.5 to 1.5 parts by weight; and
(iv) oil to form a water in oil suspension;

and
(2) polymerizing the starting components.

In such a reverse suspension process, the surfactant may be selected from the group consisting of sorbitan esters such as sorbitan monolaurate (Span® 20), sorbitan monopalmitate (Span® 40), sorbitan monooleate (Span® 80), and sorbitan trioleate (Span® 85), hydroxyethyl cellulose, mixture of glyceryl stearate and PEG stearate (Arlacel®) and cellulose acetate.

In the above processes, the polymerization initiator may include t-butyl peroxide, benzoyl peroxide, azobiscyanovaleric acid (also known as 4,4'-Azobis(4-cyanopentanoic acid)), AIBN (azobisisobutyronitrile), or 1,1' Azobis (cyclohexane carbonitrile) or one or more thermal initiators such as 2-Hydroxy-4'-(2-hydroxyethoxy)-2-methylpropiophenone (106797-53-9); 2-Hydroxy-2-methylpropiophenone (Darocur® 1173, 7473-98-5); 2,2-Dimethoxy-2-phenylacetophenone (24650-42-8); 2,2-dimethoxy-2-phenyl acetophenone (Irgacure®, 24650-42-8) or 2-Methyl-4'-(methylthio)-2-morpholinopropiophenone (Irgacure®, 71868-10-5).

Further, the oil may be selected from paraffin oil, silicone oil and organic solvents such as hexane, cyclohexane, ethyl acetate or butyl acetate.

Loading may proceed by numerous methods well-known to one of skill in the art such as passive adsorption (swelling of the polymer into a drug solution) or by ionic interaction.

In order to increase the drug loading and control the rate of drug release, a concept consists to introduce certain chemical moieties into the polymer backbone that are capable of interacting with the drug via non covalent interactions. Examples of such interactions include electrostatic interactions (described above), hydrophobic interactions, $\pi$-$\pi$ stacking, and hydrogen bonding, among others.

*Drug*

In the context of the invention, the composition comprises an effective amount of a peptides antibiotic. Peptides antibiotics, also named antimicrobial peptides, are a chemically diverse class of anti-infective and antitumor antibiotics containing non-protein polypeptide chains. Antimicrobial peptide antibiotics include in particular glycopeptide antibiotics, and polycationic peptide antibiotics.

Advantageously, the antimicrobial peptides antibiotics are glycopeptide antibiotics, in particular hydrophobic glycopeptide antibiotics, and polycationic peptides antibiotics, in particular polycationic peptides, cyclic or linear, with or without a fatty acid tail.

In the context of the invention, a "polycationic peptides" may be a cyclic peptides soluble in water, i.e. having a partition coefficient P between -2.4 (polymyxin E - colistin) and -0.9 (polymyxin B). The polymyxin scaffold contains a

hydrophobic tail, namely a methyl-octanamide chain covalently bound terminal diaminobutyric acid.

In the context of the invention, a "polycationic peptides" may also be a linear peptides soluble in water produced by different organisms (bacteria, fungi, animals and plants) usually contain a composition rich in cationic and hydrophobic amino acids, and having a cationic (positively charged) and amphiphilic characteristics, belonging to the family of cathelicidin and defensin subfamilies (Lei et al; 2019. Am J Transl Res 11:3919-31).

Advantageously, the polycationic peptides antibiotic of the invention is a polymyxin, in particular polymyxin B.

The polymyxins are cationic surfactants that act as antibiotics. Their general structure is that of a polycationic cyclic peptide with a hydrophobic tail. After interaction with the lipid A of lipopolysaccharides, a membrane destabilization occurs leading to cell death. Polymyxins have a bactericidal effect on Gram-negative pathogens, in particular Pseudomonas and Enterobacteriaceae.

In the context of the invention, a "glycopeptide antibiotic" is a glycopeptide antibiotic as defined above that is soluble in water, i.e. having a partition coefficient P of between -3.1 (vancomycin) and 4.10 (oritavancin). The glycopeptides antibiotics comprise a hydrophobic tail composed of 9 or more carbon atoms.

Advantageously, the hydrophobic glycopeptide antibiotic is selected from the group consisting of teicoplanin, vancomycin, Daptomycin, telavancin, ramoplanin, decaplanin, corbomycin, complestatin, bleomycin, oritavancin and dalbavancin, in particular teicoplanin.

Advantageously, in the composition of the invention, the antimicrobial peptides are loaded/absorbed onto the microsphere as defined above by non-covalent interactions. This particular way of entrapping drugs or prodrugs is called physical entrapment.

Loading of the antimicrobial peptides onto the microsphere of the invention may be proceeded by numerous methods well-known to the one skilled in the art such as preloading after the microsphere synthesis, or an extemporaneous loading onto preformed and sterilized microspheres

Advantageously, the composition of the invention comprises between 10 and 50 mg/ml of hydrophobic glycopeptide antibiotic, such as teicoplanin, more advantageously between 10 and 30 mg/ml.

Advantageously, the composition of the invention locally releases the hydrophobic glycopeptide antibiotic, such as teicoplanin, at a constant rate of between 100 and 500 μg.

Advantageously, the composition of the invention comprises between 2 and 10 mg/ml of polycationic peptides antibiotic, such as polymyxin B, more advantageously between 2 and 5 mg/ml.

Advantageously, the composition of the invention locally releases the polycationic peptides antibiotic, such as polymyxin B, at a constant rate of between 100 and 500 μg/day.

Advantageously, the composition of the invention releases the antimicrobial peptides so that the local concentration remains over the minimal inhibitory concentration (MIC) for 1 to 30 days, advantageously for 1 to 15 days, preferably for 1 to 7 days, the therapeutic range being between 1 ng/ml to 40 ng/ml.

*Composition*

In the context of the invention, the composition comprises an effective amount of a antimicrobial peptides, at least one hydrophilic degradable microsphere as defined above, and a pharmaceutically acceptable carrier for administration by injection.

The antimicrobial peptides and the hydrophilic degradable microsphere are as defined above.

According to the invention, the pharmaceutically acceptable carrier is intended for administration of the antimicrobial peptide by injection and is advantageously selected in the group consisting in water for injection, saline, glucose, starch, hydrogel, polyvinylpyrrolidone, polysaccharide, hyaluronic acid ester, contrast agents and plasma.

The composition of the invention can also contain a buffering agent, a preservative, a gelling agent, a surfactant, or mixtures thereof. Advantageously, the pharmaceutically acceptable carrier is saline or water for injection.

The composition of the invention allows the extended-release, in particular the controlled-release, of antimicrobial peptides over a period ranging from a few hours to a few weeks. Advantageously, the composition of the invention allows the controlled-release of antimicrobial peptides for 1 day to 14 days, advantageously for 1 day to 7 days.

The composition of the invention allows the temporal control and the sustained-release as defined above, for example by modulating the nature and the contents of monomers a), b) and/or c).

The invention also relates to the composition as defined above, for use for preventing and/or treating infectious diseases, in particular mammal infectious diseases, by local delivery.

The invention also relates to a method for preventing and/or treating infectious diseases, in particular mammal infectious diseases, comprising locally administering to a subject in need thereof an effective amount of the composition as defined above.

The invention also relates to the use of the composition as defined above for the manufacturing of a drug for preventing and/or treating infectious diseases, in particular mammal infectious diseases, by local delivery. Advantageously, the infectious diseases are caused by Gram-positive bacteria, such as methicillin-resistant *Staphylococcus*

*aureus* and *Enterococcus faecalis,* or by Gram-negative bacteria such as *Pseudomonas aeruginosa* or carbapenemase-producing *Enterobacteriaceae.*

Advantageously, the infectious diseases caused by methicillin-resistant *Staphylococcus aureus* are located in skin and soft tissues, but also the bloodstream infections, endocarditis, bone and joint infections, meningitis *P. aeruginosa* is involved in osteo-articular infections, infections of prosthetic joints, nosocomial infections, or ocular diseases such as conjunctivitis, microbial keratitis, endophthalmitis, blepharitis and dry eye. Gram-positive bacteria are the major contributor of ocular infections (Teweldemedhin et al. 2017. BMC Ophthalmology. 17:212). In keratitis diagnoses, multiple species of bacteria have been identified: *P. aeruginosa, E. coli, K. pneumoniae,* Acinetobacter, Serratia (*S. marcescens* and *S. liquefaciens*), Aeromonas, Fusobacterium, Enterobactor spp., P. mirabilis (*Proteus mirabilis*), P. multocida (*Pasteurella multocida*), M. catarrhalis (*Moraxella* catarrhalis).

*Extemporaneous loading*

In a particular embodiment of the invention, the antimicrobial peptides may be loaded extemporaneously on dry and sterile microsphere.

The invention thus also relates to a pharmaceutical kit comprising:

- i) at least one hydrophilic degradable microsphere as defined above in association with a pharmaceutically acceptable carrier for administration by injection;
- ii) an effective amount of antimicrobial peptides; and
- iii) optionally an injection device,

the hydrophilic degradable microsphere and the antimicrobial peptides being packed separately.

In such an embodiment, antimicrobial peptides is advantageously intended to be loaded on the hydrophilic degradable microsphere just before the injection.

In the context of the invention, "glycopeptide antibiotic" is advantageously a glycopeptide antibiotic as defined above that is soluble in water, i.e. having a partition coefficient P of between -3.1 (vancomycin) and 4.10 (oritavancin). The glycopeptide antibiotic comprises a hydrophobic tail composed of 9 or more carbon atoms.

Advantageously, the hydrophobic glycopeptide antibiotic is selected from the group consisting of teicoplanin, vancomycin Daptomycin, telavancin, ramoplanin, decaplanin, corbomycin, complestatin, bleomycin, oritavancin and dalbavancin.

In the context of the invention, a "polycationic peptides" may be a cyclic peptide soluble in water, i.e. having a partition coefficient P between -2.4 (polymyxin E - colistin) and -0.9 (polymyxin B). The polymyxin scaffold contains a hydrophobic tail, namely a methyl-octanamide chain covalently bound terminal diaminobutyric acid.

In the context of the invention, a "polycationic peptide" may also be a linear peptide soluble in water produced by different organisms (bacteria, fungi, animals and plants) usually contain a composition rich in cationic and hydrophobic amino acids, and having a cationic (positively charged) and amphiphilic characteristics, belonging to the family of cathelicidin and defensin subfamilies (Lei et al; 2019. Am J Transl Res 11:3919-31).

According to the present invention, "injection device" means any device for parenteral administration. Advantageously, the injection device is one or more syringes, which may be prefilled, and/or one or more catheters or microcatheters.

*Use of the microsphere*

The invention also relates to the hydrophilic degradable microsphere as defined above for use for the local delivery, advantageously the controlled and local delivery, of an effective amount of antimicrobial peptides to a subject in need thereof.

Advantageously, the sustained delivery of antimicrobial peptides is over a period ranging from a few hours to a few months without burst, advantageously from 1 day to 14 days, more advantageously from 1 day to 7 days.

The invention also relates to a method for locally delivering an effective amount of antimicrobial peptides to a subject in need thereof, advantageously over a period ranging from a few hours to a few months without burst, advantageously from 1 day to 14 days, more advantageously from 1 day to 7 days, comprising the administration of the hydrophilic degradable microsphere as defined above in association with a pharmaceutically acceptable carrier for administration by injection.

The examples which follow illustrate the invention without limiting its scope in any way.

## DESCRIPTION OF FIGURES

Figure 1 represents Polymyxin B elution after extemporaneous loading and their subsequent transfer in PBS according to example 3

## EXAMPLES

### Example 1: Preparation of unloaded microsphere according to the invention

The starting components and their contents are summarized in Table 1. Table 1 also summarizes the main parameters.

| | | Test number | MS1 | MS2 | MS3 | MS4 | MS5 |
|---|---|---|---|---|---|---|---|
| Process parameters | | Oil/Water ratio (V/V) | 1/11 | 1/11 | 1/11 | 1/11 | 1/11 |
| | | Stirring speed | 240 RPM | 240 RPM | 240 RPM | 240 RPM | 240 RPM |
| | | PVA | 1 % | 1 % | 1 % | 1 % | 1 % |
| | | NaCl | 3 % | 3 % | 3 % | 3 % | 3 % |
| Organic phase | | Monomers mass / organic phase mass (%) | 56 % | 56 % | 56 % | 56 % | 56 % |
| | | Toluene | 44 % | 44 % | 44% | 44 % | 44 % |
| | | Hexanethiol | 3 % | 3 % | 3 % | 3 % | 3 % |
| | | (% mole / PEGma mole) | | | | | |
| | | AIBN (% weight/ organic phase weight | 0.28% | 0.28% | 0.28% | 0.28% | 0.28% |
| | Phase monomer | m-PEGMA (% mole / total mole monomer) | 55 % | 51 % | 50 % | 30 % | 10 % |
| | | Crosslinker (% mole / total mole monomer) | 5 % of $PEG_{13}$-$LA_7$-$Cl_3$ | 9 % of $PEG_{13}$-$LA_7$-$Cl_3$ | 30% of $PEG_{13}$-$LA_7$-$Cl_3$ | 50 % of $PEG_{13}$-$LA_7$-$Cl_3$ | 5 % of $PEG_{13}$-$LA_4$-$Cl_5$ |
| | | 2-methylene-1,3-dioxepane (MDO) (% mole / total mole monomer) | 10 % | 10 % | 10 % | 10 % | 10 % |
| | | Methacrylic acid (% mole / total mole monomer) | 30 % | 30 % | 30 % | 30 % | 30 % |

**Table 1. Formulations of microspheres according to the invention**

The aqueous phase solution (917 mL) containing 1 % polyvinyl alcohol (Mw= 13000-23000 g/mol), 3 % NaCl in deionized water is placed in a 1 $dm^3$ reactor and heated up to 50°C.

The organic phase is prepared in an Erlenmeyer. Briefly, toluene (36.9 g) and 2,2'-azobis(2-methylpropionitrile) (AIBN) (0.28% weight/ organic phase weight) are weighted. AIBN is introduced in another vial and solubilized in a volume

fraction (≈ 30%) of the weighted toluene.

Then, degradable crosslinker is weighted in an Erlenmeyer. Polyethylene glycol methyl ether methacrylate (Mn= 300 g/mol), methacrylic acid and 2-Methylene-1,3-dioxepane (MDO) are weighted and introduced into the erlenmeyer. Then the remaining volume of toluene is added to solubilize the monomers. Hexanethiol (3 % mol /mol of PEGma) is added to the Erlenmeyer using a micropipette. The AIBN solution in toluene is added to the erlenmeyer containing monomers. Finally, the organic phase has to be clear (monomer and initiator should be totally solubilized) without any aggregates before introduction into the aqueous phase.

The organic phase is poured into the aqueous phase at 50°C. Thereupon, stirring (240 rpm) is applied by using an impeller. After 4 minutes, the temperature is raised up to 80°C. After 8 hours, the stirring is stopped and microspheres were collected by filtration on a 40 μm sieve and washed extensively with acetone and water. Microspheres were then sieved with decreasing sizes of sieves (125, 100, 50 μm).

MS in the size range 50-100 μm were collected for drug loading trials.

**Example 2: Loading of microspheres according to example 1 with teicoplanin or polymyxin B (preloading after MS synthesis)**

After the sieving step, 500 μL of microspheres obtained in example 1 (size range 50-100 μm) was placed in 15 mL polypropylene vials. Then, 0.5 up to 2 mL teicoplanin (Sigma T0578-100 mg) or 2 mL of Polymyxin B sulfate (Merck ref 5291-500MG) in water at 10 mg/mL was added to the wet microspheres. For the Polymyxin B sulfate loading, the solution was completed with 12 mM of sodium bicarbonate.

The loading step was performed at room temperature for 1 h under stirring on a tube rotator (≈ 30 rpm).

Then,

for the teicoplanin loading, the supernatants were removed for the measurement of unbound teicoplanin. Absorbance was measured at 280 nm and the amount of teicoplanin in supernatant was obtained by extrapolation from a standard curve (15 to 500 μg/mL), and the loaded dose was calculated by subtraction;

for the Polymyxin B sulfate loading, the supernatants were removed for the measurement of unbound Polymyxin B. Absorbance was measured at 220 nm and the amount of Polymyxin B in supernatant was obtained by extrapolation from a standard curve (12.5 to 500 μg/mL), and the loaded dose was calculated by subtraction.

The pellets were washed with 10 mL of a solution of glucose in water (2.5 % w/v). Then, the microsphere pellets were frozen-dried before e-beam sterilization (15 - 25 kilograys).

Table 2 summarizes loading for each MS formulation tested.

Table 2. Antibiotic loading of the microspheres according to example 2

| MS used | Antibiotics | Antibiotics loading (mg/ml) (% of loading efficacy) |
|---|---|---|
| MS1 | Teicoplanin | 15.6 (78 %) |
| MS1 | Polymyxin B | 14.3 (35%) |
| MS2 | Teicoplanin | 17.5 (87%) |

**Example 3. Extemporaneous loading of teicoplanin or polymyxin B on sterile microspheres according to example 1**

After the sieving step, a suspension of 0.5 mL or 1 mL of microspheres obtained in example 1 (size range 50-100 μm) in 15 mL of a solution containing 5 % (w/v) of glucose was prepared. After homogenization, the pellet of microspheres was recovered, frozen-dried and sterilized by e-beam radiation (15-25 kilograys).

Then, teicoplanin (1 mL or 2 mL) or polymyxin B (2 mL) in solution 10 mg/mL in water were added to the sterile and dry microspheres.

The loading step was done at room temperature for 15 minutes under stirring on a tube rotator (≈ 30 rpm). Then, the supernatants were removed for the measurement of

unbound teicoplanin, absorbance was measured at 280 nm and the amount of teicoplanin was obtained by extrapolation from a standard curve (15 to 50 μg/mL);

unbound polymyxin B, absorbance was measured at 220 nm and the amount of polymyxin was obtained by extrapolation from a standard curve (12.5 to 50 μg/mL);

Table 3 summarizes extemporaneous loading for each MS formulation tested.

Table 3. Antibiotic loading of the microspheres according to example 3

| MS used | Antibiotics | Antibiotics loading (mg/ml) (% of loading efficacy) |
|---|---|---|
| MS1 | Teicoplanin | 34 (88 %) |
| MS1 | Polymyxin B | 9.5 (47 %) |
| MS2 | Teicoplanin | 26 (73 %) |
| MS5 | Teicoplanin | 39 (97 %) |
| MS5 | Polymyxin B | 7.6 (38 %) |

Then, PBS (14 mL) was added to the microspheres loaded with teicoplanin or polymyxin B for the *in vitro* drug release experiment (37°C, 150 rpm)

**Example 4. Study of the *in vitro* release of polymyxin B from loading microsphere according to example 3**

For sterile and dry microspheres loaded polymyxin B, 14 mL of PBS (Sigma P-5368; 10 mM phosphate buffered saline; NaCl 0,136 M; KCl 0,0027 M; pH 7.4 at 25°C) were added. Drug elution occurred at 37°C under shaking (150 rpm), the tubes were placed horizontally in the oven. Samples (2 mL) were withdrawn after 10 minutes, 1h, 3h and every day until microspheres degradation. At each sampling time, the medium was completely renewed with fresh PBS.

Then, amounts of free polymyxin B in PBS supernatants were determined by RP-HPLC at 220 nm on a $C_{18}$ column (46 x 150 mm). The gradient elution was 10-60% solvent B (acetonitrile plus 0.075% TFA) over 10 min. Water containing 0.075% TFA was used as solvent A. Flow rate was 1 mL/min at 25°C. The results are shown in Figure 1.

## Claims

1. A composition comprising an effective amount of a peptides antibiotic, at least one hydrophilic degradable microsphere comprising a crosslinked matrix, and a pharmaceutically acceptable carrier for administration by injection, the crosslinked matrix being based on at least:

   a) between 10 % and 90 % of hydrophilic monomer of general formula (I):

   $$(CH_2{=}CR_1)\text{-}CO\text{-}D \qquad (I)$$

   wherein:

   - D is O-Z or NH-Z, Z being $(C_1\text{-}C_6)$alkyl group, $-(CR_2R_3)_m\text{-}CH_3$, $-(CH_2\text{-}CH_2\text{-}O)_m\text{-}H$, $(CH_2\text{-}CH_2\text{-}O)_m\text{-}CH_3$, $-C(R_4OH)m$ or $-(CH_2)_m\text{-}NR_5R_6$ with m being an integer from 1 to 30;
   - $R_1$, $R_2$, $R_3$, $R_4$, $R_5$ and $R_6$ are, independently of one another, hydrogen atom or a $(C_1\text{-}C_6)$alkyl group;

   b) between 0.1 and 30% mol of a cyclic monomer having an exo-methylene group of formula (II):

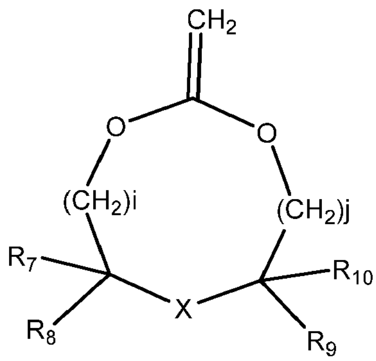

(II)

wherein:

- $R_7$, $R_8$, $R_9$ and $R_{10}$ are, independently of one another, hydrogen atom, a $(C_1-C_6)$alkyl group or an aryl group;
- i and j are independently of one another an integer chosen between 0 and 2; and
- X is a single bond or an oxygen atom;

and
c) between 5 % and 90 % of one degradable block copolymer cross-linker, wherein the degradable block copolymer crosslinker is linear or star-shaped and presents $(CH_2=(CR_{11}))$-groups at all its extremities, each $R_{11}$ being independently of one another hydrogen atom or a $(C_1-C_6)$alkyl group, and wherein the degradable block copolymer crosslinker has a partition coefficient P of between 0.50 and 11.20, or a hydrophobic/hydrophilic balance R between 1 and 20;

the percentages of the monomers a) to c) being expressed in moles relative to the total number of moles of the monomers.

**2.** The composition of claim 1, wherein the degradable block copolymer cross-linker c) is of general formula (IIIa) or (IIIb) as follows:

$(CH_2=CR_{11})\text{-CO-}X_n\text{-}PEG_p\text{-}X_k\text{-CO-}(CR_{11}=CH_2)$ (IIIa);

$(CH_2=CR_{11})\text{-CO-O-}X_n\text{-}PEG_p\text{-}X_k\text{-O-CO-}(CR_{11}=CH_2)$ (IIIb)

$PEG_p\text{-}(X_n\text{-O-CO-}(CR_{11}=CH_2))_z$ (IIIc);

wherein:

- each $R_{11}$ is independently of one another hydrogen atom or a $(C_1-C_6)$alkyl group;
- X independently represents, PLA, PGA, PLGA, PCL or PLAPCL;
- n, k and p respectively represent the degree of polymerization of X, and PEG, n and k independently being integers from 1 to 150, and p being an integer from 1 to 100;
- z represent the number of arms of the PEG molecule being integers from 3 to 8.

**3.** The composition of claim 2, wherein the degradable block copolymer cross-linker c) is of general formula (IIIa), (IIIb) or (IIIc), in particular (IIIa) or (IIIb), wherein X represents PCL or PLAPCL.

**4.** The composition of claim 2 or 3, wherein the degradable block copolymer cross-linker c) is selected from the group consisting of compounds of general formula (IIIa), (IIIb) or (IIIc), in particular (IIIa) or (IIIb), as defined above, wherein:

- X= PLA, n + k=12 and p=13;
- X= PLAPCL, n + k=10 and p=13;
- X = PLAPCL, n + k= 9 and p=13;
- X = PLAPCL, n + k= 8 and p=13;
- X = PCL; n + k = 8 and p=13;
- X =PCL, n + k = 10 and p=4; or

- X =PCL, n + k = 12 and p=2.

**5.** The composition of any one of claims 1 to 4, wherein the degradable block copolymer cross-linker c) is present in the reaction mixture in an amount of between 5% and 60 %, advantageously between 10% and 60% by mole, relative to the total number of moles of the monomers.

**6.** The composition of any one of claims 1 to 5, wherein the cyclic monomer b) is selected from the group consisting of 2-methylene-1,3-dioxolane, 2-methylene-1,3-dioxane, 2-methylene-1,3-dioxepane, 2-methylene-4-phenyl-1,3-dioxolane, 2-Methylene-1,3,6-Trioxocane and 5,6-benzo-2-methylene-1,3dioxepane.

**7.** The composition of any one of claims 1 to 6, wherein the hydrophilic monomer a) is selected from the group consisting of sec-butyl acrylate, n-butyl acrylate, t-butyl acrylate, t-butyl methacrylate, methylmethacrylate, N-dimethyl-aminoethyl(methyl)acrylate, N,N-dimethylaminopropyl-(meth)acrylate, t-butylaminoethyl (methyl)acrylate, N,N-diethylaminoacrylate, acrylate terminated poly(ethylene oxide), methacrylate terminated poly(ethylene oxide), methoxy poly(ethylene oxide) methacrylate, butoxy poly(ethylene oxide) methacrylate, acrylate terminated poly(ethylene glycol), methacrylate terminated poly(ethylene glycol), methoxy poly(ethylene glycol) methacrylate, butoxy poly(ethylene glycol) methacrylate.

**8.** The composition of any one of claims 1 to 7, wherein the crosslinked matrix of the hydrophilic degradable microsphere is further based on a chain transfer agent d).

**9.** The composition of any one of claims 1 to 8, wherein the crosslinked matrix is further based on at least one ionised or ionisable monomer d) of general formula (V):

$$(CH_2{=}CR_{12})\text{-M-E} \qquad (V),$$

wherein:

- $R_{12}$ is hydrogen atom or a $(C_1\text{-}C_6)$alkyl group;
- M is a single bond or a divalent radical having 1 to 20 carbon atoms, advantageously a single bond;
- E is a ionised or ionisable group being advantageously selected from the group consisting of - COOH, -COO-, $-SO_3H$, $-SO_3^-$, $-PO_4H_2$, $-PO_4H^-$, $-PO_4^{2-}$, $-NR_{13}R_{14}$, and $-NR_{15}R_{16}R_{17}^+$; $R_{13}$, $R_{14}$, $R_{15}$, $R_{16}$ et $R_{17}$ being independently of one another hydrogen atom or a $(C_1\text{-}C_6)$alkyl group.

**10.** The composition of any one of claims 1 to 9, wherein the peptides antibiotic is a glycopeptide antibiotic or a polycationic peptides antibiotic, advantageously the hydrophobic glycopeptide antibiotic is selected from the group consisting of teicoplanin, vancomycin, daptomycin, telavancin, ramoplanin, decaplanin, corbomycin, complestatin, bleomycin, oritavancin and dalbavancin, in particular teicoplanin, and advantageously the polycationic peptides is selected from the group of polymixins or the group of cathelicidins and defensins, in particular polymyxin B.

**11.** The composition of any one of claims 1 to 10, comprising between 10 and 50 mg/ml of a glycopeptide antibiotic, and between 2 and 10 mg/ml of a polycationic peptides antibiotic.

**12.** A composition as defined in any one of claims 1 to 11, for use for preventing and/or treating infectious diseases, in particular mammal infectious diseases, by local delivery.

**13.** A hydrophilic degradable microsphere for use for locally delivering an effective amount of a peptides antibiotic to a subject in need thereof, the hydrophilic degradable microsphere being as defined in any one of claims 1 to 11.

**14.** The hydrophilic degradable microsphere for use according to claim 13, wherein the peptide antibiotic is a glycopeptide antibiotic or a polycationic peptides antibiotic, advantageously the hydrophobic glycopeptide antibiotic is selected from the group consisting of teicoplanin, vancomycin, daptomycin, telavancin, ramoplanin, decaplanin, corbomycin, complestatin, bleomycin, oritavancin and dalbavancin, in particular teicoplanin, and advantageously the polycationic peptides is selected from the group of polymixins or the group of cathelicidins and defensins, in particular polymyxin B.

**15.** Pharmaceutical kit comprising:

i) at least one hydrophilic degradable microsphere as defined in any one of claims 1 to 11 in association with

a pharmaceutically acceptable carrier for administration by injection;
ii) an effective amount of peptides antibiotic; and
iii) optionally an injection device,

the hydrophilic degradable microsphere and the peptides antibiotic being packed separately.

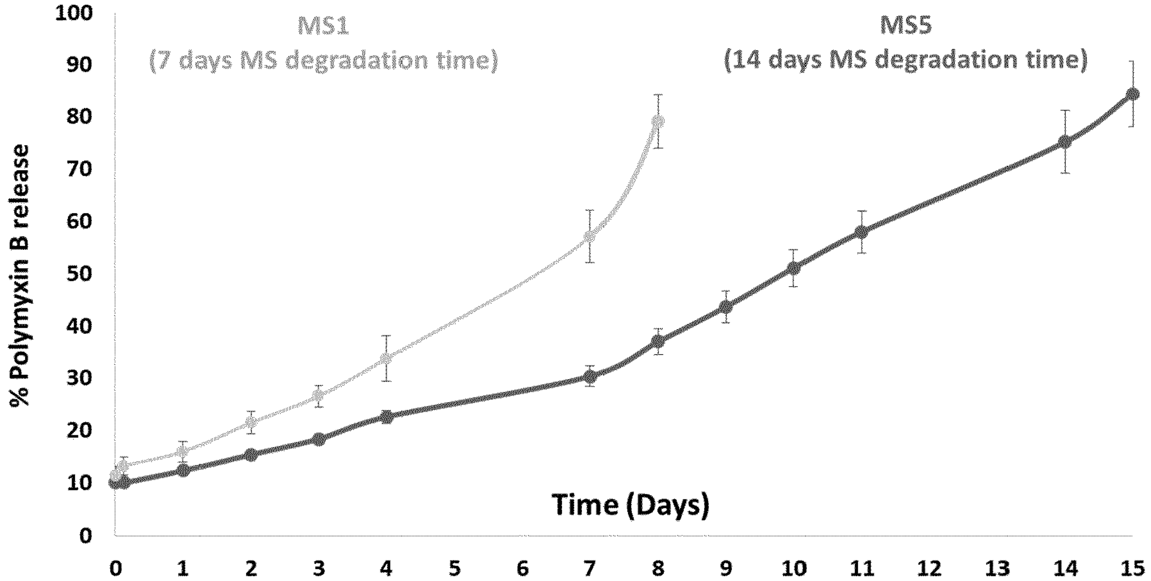
MS1
(7 days MS degradation time)
MS5
(14 days MS degradation time)
% Polymyxin B release
Time (Days)
100
90
80
70
60
50
40
30
20
10
0
0
1
2
3
4
5
6
7
8
9
10
11
12
13
14
15

Fig. 1

Europäisches Patentamt
European Patent Office
Office européen des brevets

# EUROPEAN SEARCH REPORT

Application Number
EP 20 30 5778

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | YENICE I ET AL: "In vitro/in vivo evaluation of the efficiency of teicoplanin-loaded biodegradable microparticles formulated for implantation to infected bone defects", JOURNAL OF MICROENCAPSULATION, TAYLOR AND FRANCIS, BASINGSTOKE, GB , vol. 20, no. 6 1 January 2003 (2003-01-01), pages 705-717, XP009524063, ISSN: 0265-2048, DOI: 10.3109/02652040309178082 Retrieved from the Internet: URL:https://www.tandfonline.com/doi/abs/10.3109/02652040309178082 *"Materials" on page 707* *"Preparation of PLGA (75:25) (Mw 136 000) microspheres" on page 708* *"In vivo studies" on page 709* * tables 1, 3 * ----- | 1-15 | INV. A61K9/00 A61K9/16 A61K9/19 A61K9/50 |
| Y | LI YUANYUAN ET AL: "Fabrication of Antimicrobial Peptide-Loaded PLGA/Chitosan Composite Microspheres for Long-Acting Bacterial Resistance", MOLECULES,, vol. 22, no. 10, 1 January 2017 (2017-01-01), page 1637, XP009524068, ISSN: 1420-3049, DOI: 10.3390/MOLECULES22101637 * abstract; figure 1 * ----- | 1-15 | TECHNICAL FIELDS SEARCHED (IPC) A61K |

-/--

The present search report has been drawn up for all claims

1

| Place of search | Date of completion of the search | Examiner |
|---|---|---|
| Munich | 25 November 2020 | Gerber, Myriam |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 30 5778

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WANG YUNJIAO ET AL: "Preparation, Characterization and Biological Activity of Cathelicidin-BF-30-Loaded Poly(LA-co-MA) Microspheres", JOURNAL OF NANOSCIENCE AND NANOTECHNOLOGY, AMERICAN SCIENTIFIC PUBLISHERS, US, vol. 19, no. 4, 1 April 2019 (2019-04-01), pages 2435-2442, XP009524066, ISSN: 1533-4880, DOI: 10.1166/JNN.2019.16422 * abstract * ----- | 1-15 | |
| Y | US 2018/028684 A1 (YEOMAN ROY R [US] ET AL) 1 February 2018 (2018-02-01) * paragraphs [0037], [0046] * ----- | 1-15 | |
| Y | WO 2012/120139 A1 (OCCLUGEL [FR]; CENTRE NAT RECH SCIENT [FR] ET AL.) 13 September 2012 (2012-09-13) * examples * ----- | 1-15 | TECHNICAL FIELDS SEARCHED (IPC) |
| Y | LOUGUET STÉPHANIE ET AL: "Poly(ethylene glycol) methacrylate hydrolyzable microspheres for transient vascular embolization", ACTA BIOMATERIALIA, ELSEVIER BV, NL , vol. 10, no. 3 1 January 2014 (2014-01-01), pages 1194-1205, XP009524073, ISSN: 1878-7568, DOI: 10.1016/J.ACTBIO.2013.11.028 Retrieved from the Internet: URL:https://www.sciencedirect.com/science/article/pii/S1742706113005953 [retrieved on 2013-12-07] * abstract * ----- | 1-15 | |

The present search report has been drawn up for all claims

1

| Place of search | Date of completion of the search | Examiner |
|---|---|---|
| Munich | 25 November 2020 | Gerber, Myriam |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 30 5778

This annex lists the patent family members relating to the patent documents cited in the above-mentioned European search report.
The members are as contained in the European Patent Office EDP file on
The European Patent Office is in no way liable for these particulars which are merely given for the purpose of information.

25-11-2020

| Patent document cited in search report | Publication date | Patent family member(s) | Publication date |
|---|---|---|---|
| US 2018028684 A1 | 01-02-2018 | US 10799600 B1 | 13-10-2020 |
| | | US 2018028684 A1 | 01-02-2018 |
| WO 2012120139 A1 | 13-09-2012 | AU 2012224525 A1 | 26-09-2013 |
| | | EP 2683753 A1 | 15-01-2014 |
| | | ES 2601211 T3 | 14-02-2017 |
| | | JP 6063876 B2 | 18-01-2017 |
| | | JP 2014511427 A | 15-05-2014 |
| | | US 2013344160 A1 | 26-12-2013 |
| | | WO 2012120139 A1 | 13-09-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KANELLAKOPOULOU K et al.** *Drugs,* 2000, vol. 59, 1223-32
- **MEEKER et al.** *J Arthroplasty,* 2019, vol. 34, 1458-61
- **SORENSEN TS et al.** *Acta Orthop Scand.,* 1990, vol. 61, 353-6
- **VELKOV et al.** *Future Med Chem.,* 2016, vol. 8, 1017-25
- **POIREL et al.** *Clin Microbiol Rev.,* 2017, vol. 30, 557-96
- **STEGMAYR.** *Ther Apher.,* 2001, vol. 5, 123-7
- **NANJO et al.** *Journal of Infection and Chemotherapy,* 2013, vol. 19, 683-90
- **MUNITA et al.** *Clin Infect Dis.,* 2015, vol. 61 (2), S48-S57
- **TRUEBA et al.** *J Clin Microbiol.,* 2006, vol. 44, 1922-23
- **JIA et al.** *Acta Biomaterialia,* 2010, vol. 6, 812-19
- **PENG et al.** *Biomaterials,* 2010, vol. 31, 5227-36
- **JIA et al.** *Antimicrob Agents Chemother.,* 2010, vol. 54, 170-176
- **YENICE et al.** *Journal of Microencapsulation,* 2003, vol. 20, 705-17
- **KAHDESTANI et al.** *Polym. Bull.,* 2020, 20
- **DROGNITZ et al.** *Infection,* 2006, vol. 34 (1), 29-34
- **LEI et al.** *Am J Transl Res,* 2019, vol. 11, 3919-31
- **TEWELDEMEDHIN et al.** *BMC Ophthalmology,* 2017, vol. 17, 212